# EUROPÄISCHE PATENTSCHRIFT

(11) **EP 0 248 215 B1**
(45) Veröffentlichungstag und Bekanntmachung des Hinweises auf die Patenterteilung: **07.07.1993**
(21) Anmeldenummer: 87106390.5
(22) Anmeldetag: 02.05.1987
(51) Int. Cl.: A61K 35/78

(54) **Arzneimittel mit dopaminerger Wirkung**
Medicine with a dopaminergic activity
Médicament à action dopaminergique

(30) Priorität: 03.06.1986 DE 3618627
(43) Veröffentlichungstag der Anmeldung: 09.12.1987
(73) Patentinhaber: Apotheker Popp oHG, D-90480 Nürnberg (DE)
(72) Erfinder: Popp, Hans Oskar, Dr. med., D-8500 Nürnberg 50 (DE)
(74) Vertreter: Matschkur, Götz, Lindner Patent- und Rechtsanwälte

(56) Entgegenhaltungen:
- CHEMICAL ABSTRACTS, Band 71, 1969, Seite 300, Zusammenfassung Nr. 73980v, Columbus, Ohio, US; L. DE CAPITE: "Histology, anatomy, and antibiotic properties of Vitex agnuscastus", & ANN. FAC. AGR. UNIV. STUDI PERUGIA 1967, 22, 109-26
- CHEMICAL ABSTRACTS, Band 71, Nr. 16, 20. Oktober 1969, Seite 423, Zusammenfassung Nr. 106289g, Columbus, Ohio, US; K. GOERLER et al.: "Iridoid derivatives from Vitex agnus-castus", & PLANTA MED 1985, (6), 530-1
- ROTE LISTE 1985, no. 45167

## Beschreibung

Die Erfindung richtet sich auf die Verwendung eines Mittels zur Herstellung eines Arzneimittels zur Behandlung der Hyperprolaktinämie.

Dopamin ist als Neurotransmitter bekannt. Diese Substanz überträgt Informationen zwischen Nervenzellen. Es kommt vor im wesentlichen im Corpus striatum, Putamen und Nucleus caudatus. Abweichungen von der normalen Konzentration, aber auch der Relation verschiedener Transmitter zueinander sind vielfach ursächlich für bestimmte Krankheitserscheinungen. So stehen im Striatum normalerweise Dopamin und Acetylcholin als dopaminerges und cholinerges System im Gleichgewicht. Folge eines Dopaminmangels oder Überwiegen des Acetylcholins ist beispielsweise der Morbus Parkinson. Die Therapie besteht dann darin, das natürliche Gleichgewicht beider Systeme wieder herzustellen. Bevorzugt bedient man sich hierzu des L-Dopa, einer Vorstufe des Dopamin, die ein bestehendes Ungleichgewicht zu Gunsten von Dopamin zu verschieben geeignet ist. Hierbei auftretende Nebenwirkungen veranlaßten bei der Behandlung des Morbus Parkinson zum Einsatz von Substanzen, die keine Dopaminstruktur aufweisen, jedoch die Fähigkeit besitzen, die Dopamin-Rezeptoren zu besetzen und damit zu stimulieren, sogenannte Dopamin-Agonisten bzw. dopaminerge Substanzen. Bekanntester Dopamin-Agonist ist das Bromocriptin.

Unter physiologischen Bedingungen kommt Dopamin eine entscheidende Bedeutung zur Steuerung der Inkretion des Prolaktin zu, welches ein hypophysäres Hormon ist, dessen Inkretion der Kontrolle des Hypothalamus unterliegt. Diese hypothalamischen Neurohormone, nämlich ein hemmendes PIF (Prolaktin inhibiting Factor) und ein stimulierendes, noch nicht näher bekanntes Releasing-Hormon TRH erreichen über das Portalsystem den Hypophysen-Vorderlappen, in deren laktotropen Zellen das Prolaktin gebildet wird. Der hemmenden Komponente kommt nach den bisherigen Erkenntnissen bei der Steuerung der Prolaktin-Ausschüttung die entscheidende Bedeutung zu. Steht nicht ausreichend Dopamin (vermutlich identisch mit PIF) zur Inhibierung der Prolaktin (PRL) - Ausschüttung zur Verfügung, ergibt sich eine erhöhte PRL-Konzentration im Blut, die sogenannte Hyperprolaktinämie. Diese Erscheinung ist ursächlich für die verschiedensten Krankheitssymptome. So kann es bei der Frau zur Corpus-luteum-Insuffizienz mit der Entwicklung eines Prämenstruellen Syndroms, zur Mastodynie, Mastopathie, Blutungsstörungen, Sterilität und bis zur Amenorrhoe kommen. Beim Mann kann die Hyperprolaktinämie eine Abnahme der Libido und Potenz, Sterilität und auch Akne hervorrufen. Bei einer Überproduktion an Prolaktin bis zur Enthemmung der Prolaktininkretion, die die verschiedensten hier nicht zu erörternden Ursachen haben können, kommt den Prolaktinhemmern eine entscheidende Bedeutung zu. Mit dopaminergen Prolaktinsenkern wurden bereits große therapeutische Erfolge erzielt. In erster Linie kommt hier die Gruppe der Mutterkornderivate mit Bromocriptin als Prototyp in Betracht. Auch eine Therapie mit L-Dopa hat Erfolge gezeigt. Nicht unerhebliche Nebenwirkungen dieser Behandlung wie z.B. Übelkeit, Erbrechen, Schwindel, Halluzinationen u.a. lassen es angezeigt erscheinen, nach anderen wirksamen, jedoch weniger nebenwirkungsreichen dopaminergen Substanzen zu suchen. Bei diesen Arbeiten zum Aufsuchen eines Arzneimittels mit dopaminerger Wirkung zur Behandlung von Krankheiten, die - ganz oder teilweise - durch einen Dopamin-Mangel verursacht oder beeinflußt werden, ist man erfindungsgemäß auf die Droge vitex agnus castus gestoßen, deren Auszüge sich als außerordentlich wirksam als Dopaminagonist erwiesen haben.

Agnus castus gehört zur Familie der Verbenaceen und ist ein im Mittelmeergebiet und in Asien beheimateter Strauch, dessen reife getrocknete und zerkleinerte Früchte offensichtlich Stoffe enthalten, die eine dopaminerge Wirkung entfalten. Die Eignung des agnus castus als wirksames Lactagogon wurde ebenso beschrieben wie bereits im Altertum die Wirkung dieser Pflanze auf die Sexualsphäre bekannt war. Sie wurde auch bereits als Aphrodisiacum genannt. Nach diesen Beobachtungen geht man von einer hormonähnlichen Wirkung der Pflanze aus. Bei einem Phytotherapeutikum wird die Wirkung der Inhaltsstoffe des vitex agnus castus auf die Stimulierung der Corpus-Luteum-Bildung und eine kreislaufanregende Eigenschaft genutzt.

Aus der Beschreibung eines vorbekannten auf den Wirkstoffen aus vitex agnus castus beruhenden Arzneimittels geht hervor, daß dadurch die Produktion des follikelstimulierenden Hormons im Vorderlappen der Hypophyse gehemmt, die Ausschüttung von luteinisierendem und luteomammotropem Hormon vermehrt, also FSH gebremst, hingegen LH und LMTH (= Prolaktin) gefördert werde. Diese Literatur geht von einer günstigen Wirkung von agnus castus bei Behandlung der Hypogalaktie aus. Durch dieses Präparat soll die Stilleistung gesteigert werden, was gleichbedeutend ist mit einer Erhöhung des Prolaktinspiegels.

Auf der Suche nach einem Mittel mit besonderer Wirkung der die Prolaktinbildung hemmenden Komponente weist die Erfindung auf eine neue Indikation für die Verwendung von agnus castus hin, nämlich auf die Verwendung von Extrakten der Pflanze vitex agnus castus zur Behandlung der Hyperprolaktinämie. Zum Nachweis der Wirksamkeit von vitex agnus castus als Prolaktinhemmer kommen wäßrige wie vor allem alkoholische Auszüge in Betracht. Die dopaminerge Wirkung von agnus castus wurde im in-vitro-Modell, in der Hypophysenzellkultur von Ratten dadurch nachgewiesen, daß der basale und der TRH-stimulierte Prolaktinspiegel unter der Gabe von agnus castus vermindert werden konnte. Von besonderer Bedeutung ist, daß das erfindungsgemäß verwendete Mittel bereits nach peroraler Applikation seine dopaminerge Wirkung entfaltet.

Beispielsweise werden die folgenden Herstellungsverfahren zur Gewinnung eines wirksamen Extraktes genannt:

### Beispiel 1

### Tinctura Agni casti (HAB 1)

Verwendet werden die reifen, getrockneten und zerkleinerten Früchte von Vitex Agnus castus L. Ein Teil der nach Vorschrift zerkleinerten Droge wird mit 0,5 Teilen Ethanol 62 % G/G gleichmäßig durchfeuchtet und bleibt mindestens zwei Stunden lang bedeckt stehen. Dann wird abgesiebt (Sieb 2) und die aus Droge und Alkohol bestehende Masse in einen Perkolator gefüllt und die Drogenoberfläche mit Filterpapier abgedeckt. Mit der erforderlichen Menge an Extraktionsflüssigkeit bis zu einem Verhältnis von einem Teil Droge zu 10 Teilen Ethanol wird der Perkolator danach aufgefüllt. Der Perkolator wird bedeckt und bleibt 24 Stunden stehen. Der Ablauf der Extraktionsflüssigkeit wird so eingestellt, daß für 100 g Droge vier bis sechs Tropfen pro Minute abtropfen. Nach Beendigung des Abtropfens wird der Drogenrückstand ausgepreßt, die Flüssigkeit mit dem Perkolat vereinigt und die Mischung filtriert.

### Beispiel 2

### Tinctura Agni casti (DAB 8)

Verwendet werden die reifen, getrockneten und zerkleinerten Früchte von Vitex Agnus castus L. Die Extraktion der Früchte erfolgt durch Perkolation im Verhältnis von einem Teil Droge zu 10 Teilen Extraktionsmittel 62 % G/G Ethanol.

Die ausgenutzte Höhe (Länge des Drogendochtes) des Perkolators beträgt mindestens das 5-fache des mittleren Durchmessers des Gefäßes.

Die vorschriftsmäßig zerkleinerte Droge wird mit der Menge der vorgeschriebenen Flüssigkeit, die 30 % des Drogengewichtes entspricht, gleichmäßig durchfeuchtet und bleibt mindestens zwei Stunden lang bedeckt stehen. Dann wird abgesiebt (2800) und die Droge unter schwachem Druck bei geöffnetem Abflußhahn in den unten mit einer Watteschicht verschlossenen Perkolator eingefüllt. Die Drogenoberfläche wird z.B. durch Filtrierpapier oder Glaskugeln so abgedeckt, daß beim Nachgießen der Flüssigkeit keine Drogenteile aufgewirbelt werden. Es wird langsam so viel Extraktionsflüssigkeit zugegeben, bis die Extraktlösung abzutropfen beginnt. Bei geschlossenem Hahn wird so viel nachgefüllt, daß die Oberfläche der Droge mit Flüssigkeit bedeckt ist. Der Perkolator wird bedeckt und bleibt 24 Stunden lang stehen. Danach läßt man die Flüssigkeit derart abfließen, daß für je 100 g Droge 4 bis 6 Tropfen in der Minute abtropfen. Die Extraktionsflüssigkeit wird so nachgegossen, daß die Drogenoberfläche stets bedeckt bleibt. Ist nach Beendigung der Zugabe die im Perkolator noch vorhandene Extraktionsflüssigkeit abgetropoft, wird der Drogenrückstand ausgepreßt und die Mischung filtriert.

### Nachweis der dopaminergen Wirkung

Der Nachweis der dopaminergen Wirkung von alkoholischen Auszügen aus Agnus castus erfolgte am pharmakologischen Modell der Prolaktin-Produktion bzw. -Freisetzung in einer Zellkultur aus Rattenhypophysen nach Spona et al. (Spona, J.; Müntz, R.; Nicolics, K.; Seprödi, J.; Teplan, J.: Action of Inhibitory LH-RH Analogs in Rat Pituitary and Luteal Cell Cultures. Endocrinologia Experimentalis 18, 101-107, 1984) gemäß folgender Versuchsanordnung:

### Herstellung der Hypophysen-Zellkultur:

Weibliche Spraque-Dowley-Ratten mit einem Gewicht von 150 -200 g wurden für eine primäre Hypophysen-Zellkultur benötigt. Die Hypophysen wurden herausgelöst und nach Entfernung der Hinterlappen in eine sterile, ausgewogene Salzlösung gegeben (Zusammensetzung: 0,8 % NaCl, 0,02 % KCl, 0,1 % NAHCO₃, 0,2 % Glucose, Einstellung des pH auf 7,2 mit NaH₂PO₄). Nach Spülung wurden 40 - 50 Hypophysenvorderlappen mit 0,25 % Trypsin bei Raumtemperatur 20 min lang vorbehandelt (Sigmatype III, Sigma-Chemicals St. Louis, Mo. U.S.A.). Nach Entfernung der ersten Trypsinlösung wurden 20 ml frische Trypsinlösung zugegeben und die Suspension über Nacht bei 0 - 4 °C stehen gelassen. Die Trypsinierung wurde durchgeführt 20 min lang bei 37 °C in 5 % CO₂-Atmosphäre unter Umrühren mit einem Teflon-Rührstab (500 Upm). Die Zellsuspension wurde in gekühlte Zentrifugenröhrchen übertragen, danach unverzüglich 5 ml einer 1 % Sojabohnen-Trypsin-Inhibitor-Lösung (Sigma-Chemicals) dazugegeben. Die Zellen wurden 15 min zentrifugiert mit 800 xg bei 4 °C in einer Beckmann-J-6-Zentrifuge (Beckmann Instruments, Palo Alto, Ca, U.S.A.). Das abgetrennte Zellmaterial wurde 3 mal gewaschen durch Aufschwemmung im Medium HAM F-12, das 50 gml⁻¹ Streptomycin und 50 Uml⁻¹ Penicillin (Difco, U.S.A.) enthält, und wiederholt zentrifugiert. Nach erneuter Aufschwemmung im Medium und Filtration durch ein Edelstahlsieb wurden die Zellen mit Trypanblau gefärbt und gezählt. Die Suspension wurde auf 6 × 105 Zellen/ml verdünnt und 3 ml davon wurden jeweils in Gewebekultur-Zylinder (25 cm² Oberfläche, Costar, U.S.A.) gegeben. CR-Transferrin (5 ngml⁻¹), CR-Fibroblasten-Wachstumsfaktor (1 ngml⁻¹), CR-Multiplikation stimulierende Aktivität (1 ngml⁻¹), Selensäure (6,4 ngml⁻¹) und 0,5 mgml⁻¹ L-Glutamin wurde dem Medium zugeführt. Die Zylinder wurden bei 37 °C unter 5 % CO₂-Atmosphäre und maximaler Feuchtigkeit aufbewahrt. Nach zwei Tagen wurde das Kulturmedium ausgetauscht.

Nach drei oder vier Tagen zeigte das Zellkultursystem die stärkste Antwort auf eine TRH-Stimulation.

### Durchführung der Versuche

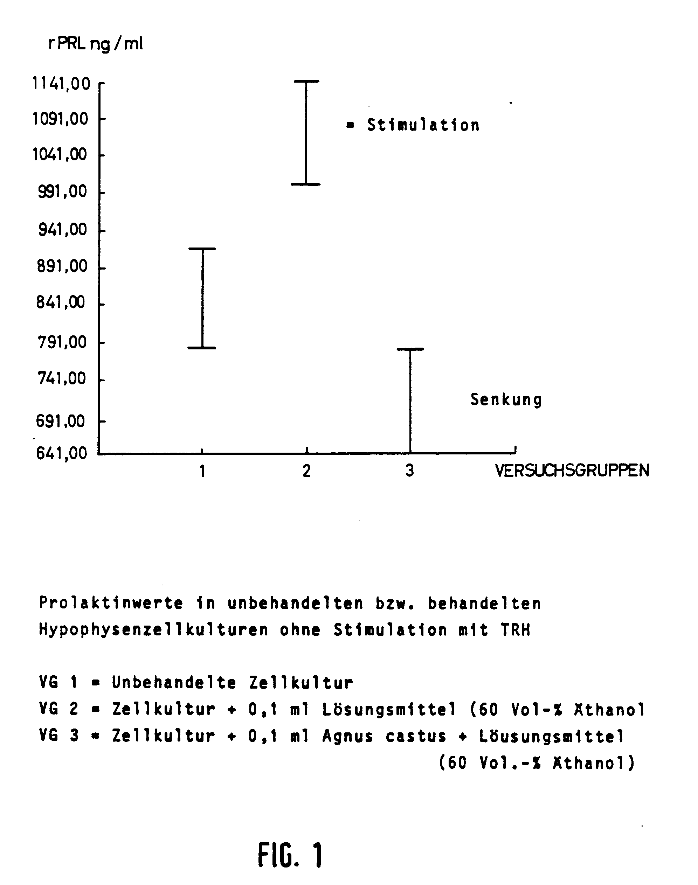

### Basale Prolaktinwerte (Fig. 1)

1. Ohne Zugabe von Stoffen (unbehandelte Zellkultur)
   Aus elf Zellkulturen wurde je 0,4 ml Medium entnommen und das basale Prolaktin bestimmt.
2. Zugabe von Lösungsmittel
   In neun Zellkulturen wurde jeweils 0,1 ml Äthanol 60 Vol-% zugegeben. Nach Inkubation von vier Stunden wurde je 0,4 ml Medium entnommen und das Prolaktin bestimmt.
3. Zugabe von Agnus castus
   In elf Zellkulturen wurde jeweils 0,1 ml Agnus castus sowie 0,1 ml Äthanol 60 Vol% zugegeben. Nach Inkubation von vier Stunden wurde je 0,4 ml Medium entnommen und das Prolaktin bestimmt.

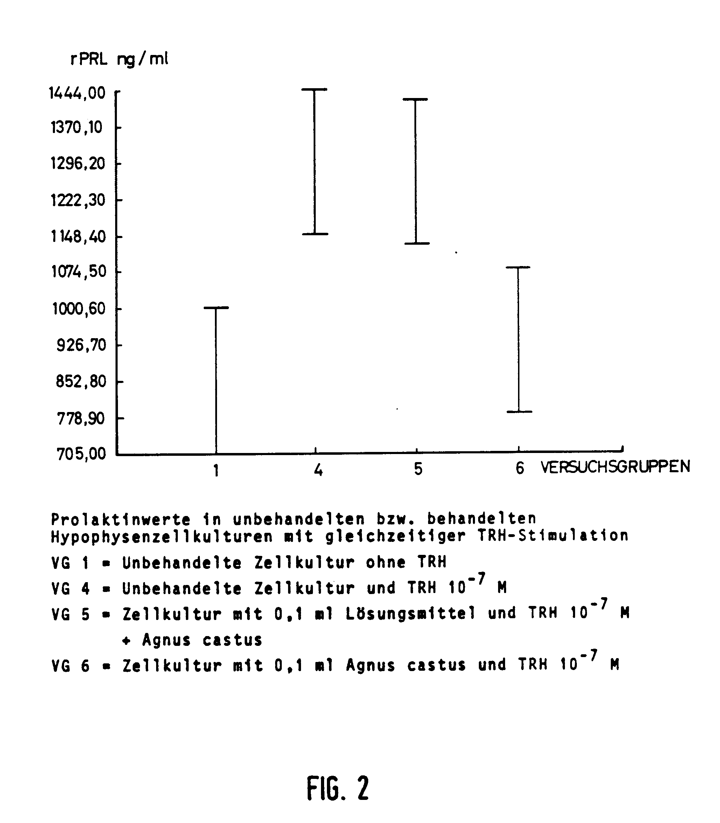

### Stimulierte Prolaktinwerte (Fig. 2)

4. Unbehandelte Zellkultur
5. Zugabe von TRH
   In elf Zellkulturen wurde jeweils 10⁻⁷M TRH zugegeben. Nach Inkubation von vier Stunden wurde je 0,4 ml Medium entnommen und das Prolaktin bestimmt.
6. Zugabe von TRH und Äthanol
   In neun Zellkulturen wurden jeweils 10⁻⁷ M TRH und 0,1 ml 60 Vol-% Äthanol gegeben. Nach Inkubation von vier Stunden wurde je 0,4 ml Medium entnommen und das Prolaktin bestimmt.
6. Zugabe von TRH und Agnus castus
   In 10 Zellkulturen wurden jeweils 10⁻¹ M TRH, 0,1 ml 60 Vol-% Äthanol und 0,1 ml Agnus castus zugegeben. Nach Inkubation von vier Stunden wurde je 0,4 ml Medium entnommen und das Prolaktin bestimmt.

### Prolaktinbestimmung

Prolaktin, das ins Medium abgegeben wurde, wurde mit einer Doppelantikörper-Methode radioimmunologisch bestimmt. Das Material für die radioimmunologische Bestimmung stammte von dem "National Institute of Arthritis, Diabetes and Digestive and Kidney Diseases (NIADDK).

Der dopaminerge Einfluß von alkoholischen Auszügen aus vitex agnus castus auf die basale Bildung und/oder Freisetzung von Prolaktin geht aus Fig. 1 hervor. Die Werte der Versuchsgruppe 2 als Kontrollgruppe liegen signifikant (Varianzanalyse; Scheffé-Test: p < 0,05) über den Werten der unbehandelten Zellkultur der Gruppe 1.

Die Werte der Versuchsgruppe 3(Inkubation mit 0,1 ml agnus castus) liegen wiederum signifikant (Varianzanalyse; Scheffè-Test: p < 0,05) unter denen der Gruppe 2 (Lösungsmittël-Kontrollgruppe) und der unbehandelten Versuchsgruppe 1.

Der dopaminerge Einfluß der alkoholischen Auszüge von vitex agnus castus auf die TRH-stimulierte Bildung und/oder Freisetzung von Prolaktin ergibt sich aus Fig. 2.

Die Werte der Gruppe 4 (Stimulation mit 10⁻⁷ M TRH) sind signifikant (Varianzanalyse; Scheffé-Test: p < 0,01) höher als die der Versuchsgruppe 1 (Basalwerte).

Die Werte der Gruppe 5 (Stimulation mit 10⁻⁷ M TRH und 0,1 ml 60 Vol-% Äthanol) sind signifikant (Varianzanalyse; Scheffè-Test: p< 0,01) höher als die der Gruppe 1(Basalwerte). Die Werte der Versuchsgruppe 5 unterscheiden sich nicht wesentlich von denen der Versuchsgruppe 4.

Die Werte der Gruppe 6 (Stimulation mit 10⁻⁷ M TRH und Inkubation mit 0,1 ml agnus castus sind signifikant (Varianzanalyse; Scheffè-Test. p < 0,01) niedriger als die der Gruppen 4 und 5. Sie unterscheiden sich nicht wesentlich von denen der Gruppe 1.

Daraus folgt, daß die Einwirkung von alkoholischen Auszügen aus vitex agnus castus auf die Produktion und/oder Freisetzung des basalen und stimulierten Prolaktins zu einer Hypophysen-Zellkultur (3) zu einer signifikanten Verringerung der Prolaktinwerte im Vergleich zu einer unbehandelten (1) und mit Lösungsmitteln (2) behandelten Hypophysen-Zellkultur führt. (vgl. Fig. 1).

Bei gleichzeitiger Gabe von agnus castus und TRH (6) zu einer Hypophysenzellkultur wird die Stimulation der Prolaktinwerte durch TRH vollständig gehemmt. Die Prolaktinwerte dieser Gruppe (6) sind signifikant geringer als die der mit TRH (4) bzw. mit TRH und Lösungsmittel (5) behandelten Versuchsgruppen (vgl. Fig. 2).

## Patentansprüche

1. Verwendung von Extrakten der Pflanze vitex agnus castus zur Herstellung eines Arzneimittels zur Behandlung der Hyperprolaktinämie.

## Claims

1. Use of extracts of the plant Vitex agnus castus for manufacturing a drug for the treatment of hyperprolactinaemia.

## Revendications

1. Utilisation d'extraits de la plante vitex agnus castus pour préparer un médicament destiné au traitement de l'hyperprolactinémie.
